# EUROPEAN PATENT APPLICATION

(11) **EP 4 516 211 A1**
(43) Date of publication of application: **05.03.2025**
(21) Application number: 24807580.6
(22) Date of filing: 16.05.2024
(51) Int. Cl.: A61B 5/00, A61B 5/11, A61B 5/103, G16H 50/20, G16H 50/30

(54) **BEDSORE RISK EVALUATION SYSTEM AND METHOD THEREOF**

(30) Priority: 16.05.2023 KR 20230063367
(71) Applicant: Neo Able Co.,Ltd., Wonju-si Gangwon-do (KR)
(72) Inventor: LEE, Myung Won, Seoul 05003 (KR); BAEK, Seung Yeob, Gunpo-si Gyeonggi-do 15875 (KR)
(74) Representative: IPrime Rentsch Kaelin AG
(86) International application number: PCT/KR2024/006673
(87) International publication number: WO 2024/237709

(57) **Abstract**

A system for evaluating a bedsore risk includes a pressure data reception unit that receives pressure data from a plurality of pressure sensors, a posture and body portion estimation unit that detects the coordinates of key points that are feature points of a body and estimate a posture based on pressure data, to output coordinate data of a pressure region of interest (PROI), that is, an interested pressure region, based on the coordinates of the key points and the posture, and that estimates a body portion, a by-portion risk evaluation unit that evaluates a degree of risk for each body portion based on the coordinate data of the PROI, statistical data, and learning data and to generate by-portion risk evaluation data, and a by-portion risk evaluation data output unit that outputs the by-portion risk evaluation data by dividing the by-portion risk evaluation data into grades according to bedsore occurrence risks.

## Description

### BACKGROUND

### 1. Technical Field

The present disclosure relates to a system and method for evaluating a bedsore risk, and more particularly, to a system and method for evaluating a bedsore risk, which can estimate a posture and a body portion based on pressure data, evaluate a bedsore risk for each estimated body portion, and provide intuitive data based on bedsore risk evaluation data.

### 2. Related Art

A circulatory disorder may appear in a body portion that is pressured continuously and repeatedly. In particular, skin damage attributable to ischemia of skin, subcutaneous fat, or muscles may occur as oxygen and nutrition to be supplied to a capillary falls short due to pressure that is applied to a projection part of a bone of the human body. There is a good possibility that bedsores will occur in a skin portion of the elderly, the disabled, or a patient who cannot control himself or herself, which comes into direct contact with a floor, because the elderly, the disabled, or the patient maintains the same posture for a long period of time. In order to prevent such bedsores, it is necessary to solve pressure that is applied to a body portion by changing the posture at predetermined time intervals and to maintain the body portion drily.

Conventionally, in order to prevent bedsores, a person who takes action, such as medical personnel and care workers, identifies a portion of a patient at which pressure occurs and performs activities to solve pressure that is applied to the portion. However, such a method requires a lot of time and costs because a person who takes action has to manually change the posture of a patient in order to solve pressure that is applied to a body of the patient. Furthermore, there is a limitation in that a person who takes action continuously and consistently determines a degree of risk of the occurrence of bedsores all day.

Furthermore, the risk of developing bedsores needs to be managed for each subject because pressure that is applied to a portion is different depending on personal conditions, such as weight, a height, sex, and an age, even if the portion is the same portion. However, it is difficult for a person who takes action to check pressure that is applied to each portion according to a posture of a subject one by one and to evaluate a degree of risk of the occurrence of bedsores.

Accordingly, there is a need to develop a system for evaluating a bedsore risk, which can check pressure that is applied to each body portion depending on a posture for each subject, determine a bedsore risk, and provide intuitive data for a change into a correct posture in order to prevent bedsores.

### SUMMARY

Various embodiments are directed to providing a system and method for evaluating a bedsore risk, which can estimate a posture and a body portion based on pressure data, evaluate a bedsore risk for each estimated body portion, and provide intuitive data based on bedsore risk evaluation data.

Furthermore, various embodiments are directed to providing a system and method for evaluating a bedsore risk, which can detect the coordinates of key points, that is, feature points of a body, by calculating the depth of pressure data.

Furthermore, various embodiments are directed to providing a system and method for evaluating a bedsore risk, which can estimate a body portion according to a posture by activating key points having coordinates, which are required for each posture, and extracting the coordinate data of a pressure region of interest (PROI), that is, an interested pressure region, on the basis of the coordinates of the activated key points.

Furthermore, various embodiments are directed to providing a system and method for evaluating a bedsore risk, which can output risk evaluation data customized for a user by evaluating a degree of risk for each body portion based on extracted coordinate data of a PROI and incorporating statistical data and learning data.

Furthermore, various embodiments are directed to providing a system and method for evaluating a bedsore risk, which can provide intuitive auxiliary data by generating three-dimensional (3-D) data based on pressure data and the coordinate data of a PROI and providing the 3-D data.

In an embodiment, a system for evaluating a bedsore risk may include a pressure data reception unit configured to receive pressure data from a plurality of pressure sensors, a posture and body portion estimation unit configured to detect the coordinates of key points that are feature points of a body and estimate a posture based on pressure data, to output coordinate data of a pressure region of interest (PROI), that is, an interested pressure region, based on the coordinates of the key points and the posture, and to estimate a body portion, a by-portion risk evaluation unit configured to evaluate a degree of risk for each body portion based on the coordinate data of the PROI, statistical data, and learning data and to generate by-portion risk evaluation data, and a by-portion risk evaluation data output unit configured to output the by-portion risk evaluation data by dividing the by-portion risk evaluation data into grades according to bedsore occurrence risks.

The posture and body portion estimation unit may include a posture estimation unit configured to store a posture estimation model that outputs the coordinates of the key points and the posture by using the pressure data as an input, and a body portion estimation unit configured to store a body portion estimation model that outputs the coordinate data of the PROI by using the coordinates of the key points and the posture as an input.

The posture estimation model may calculate a depth based on a pressure intensity of the pressure data, and may detect the coordinates of the key points in a three-dimensional (3-D) space based on the calculated depth.

The body portion estimation model may activate the key point having coordinates, which is required for each posture, may generate a clipping mask on the basis of the coordinates of the activated key point, and may extract the coordinate data of the PROI by projecting the generated clipping mask onto the pressure data.

The by-portion risk evaluation unit may output the by-portion risk evaluation data by assigning an accumulation weight over time to the by-portion risk evaluation data. The accumulation weight may include at least one of an accumulated time, the presence or absence of bedsores, a nutrition state, or a skin state.

The system may further include a 3-D data generation unit configured to generate and provide the pressure data as 3-D data based on the coordinate data of the PROI. The generated 3-D data may be supplemented by using pre-stored posture image data and be displayed by incorporating the by-portion risk evaluation data into the supplemented 3-D data.

Furthermore, in an embodiment, a method of evaluating a bedsore risk may include steps of a) receiving pressure data from a plurality of pressure sensors, b) detecting coordinates of key points that are feature points of a body and estimating a posture by inputting the pressure data to a posture estimation model, c) outputting coordinate data of a pressure region of interest (PROI) that is an interested pressure region by inputting the coordinates of the key points and the posture to a body portion estimation model and estimating a body portion, and d) evaluating a degree of risk for each body portion and generating by-portion risk evaluation data based on the coordinate data of the PROI, statistical data, and learning data and outputting the by-portion risk evaluation data by dividing the by-portion risk evaluation data into grades according to bedsore occurrence risks.

The step b) may include calculating a depth based on a pressure intensity of the pressure data and detecting the coordinates of the key points in a three-dimensional (3-D) space based on the calculated depth.

The step c) may include activating a key point having coordinates, which is required for each posture, generating a clipping mask on the basis of coordinates of the activated key point, and extracting the coordinate data of the PROI by projecting the generated clipping mask onto the pressure data.

The step d) may include outputting the by-portion risk evaluation data by assigning an accumulation weight over time to the by-portion risk evaluation data. The accumulation weight may include at least one of an accumulated time, the presence or absence of bedsores, a nutrition state, or a skin state.

The method may further include a step e) of generating and providing pressure data as 3-D data based on the coordinate data of the PROI. The step e) may include supplementing the generated 3-D data by using pre-stored posture image data and displaying the supplemented 3-D data by incorporating the by-portion risk evaluation data into the supplemented 3-D data.

According to the embodiments of the present disclosure, a posture and a body portion can be estimated based on pressure data. A bedsore risk can be evaluated for each body portion according to an estimated posture. That is, pressure that is applied to the body of a subject can be accurately checked and solved because a bedsore risk of each body portion can be evaluated for each posture.

Furthermore, according to the embodiments of the present disclosure, the coordinates of key points, that is, feature points of a body, can be detected based on pressure data. Furthermore, a posture of a subject can be estimated more accurately because the coordinates of the key points have been preset for each posture.

Furthermore, according to the embodiments of the present disclosure, key points having coordinates, which are required for each posture, can be activated. A clipping mask can be generated on the basis of the coordinates of the activated key points. Furthermore, the coordinate data of a PROI, that is, an interested pressure region, can be obtained by projecting a clipping mask onto pressure data. In this case, a customized interested pressure region can be checked and a body portion region can be estimated by incorporating body characteristics of a subject because the size of the clipping mask is adjusted depending on conditions, such as the height and weight of the subject.

Furthermore, according to the embodiments of the present disclosure, personalized risk evaluation data for each body portion can be output by incorporating the coordinate data of a PROI, learning data, and statistical data.

Furthermore, according to the embodiments of the present disclosure, 3-D data can be generated and provided based on pressure data and the coordinate data of a PROI. In this case, inaccurate pressure data of an untouched portion of a body can be supplemented based on pre-stored posture image data. Furthermore, a person who takes action can intuitively check a bedsore risk through a 3-D image and rapidly take measures against the bedsore risk by incorporating risk evaluation data for each body portion into the generated 3-D data.

Effects of the present disclosure are not limited to the above effects, and may be understood as including all effects which may be inferred from the description of the present disclosure or a construction of the invention described in the claims.

### BRIEF DESCRIPTION OF THE DRAWINGS

FIG. 1 is a diagram illustrating a construction of a system for evaluating a bedsore risk according to an embodiment of the present disclosure.
FIG. 2 is a diagram illustrating an operation of a posture and body portion estimation unit according to an embodiment of the present disclosure.
FIG. 3 is a diagram illustrating that a body portion estimation unit outputs the coordinate data of a PROI according to an embodiment of the present disclosure.
FIG. 4 is a diagram illustrating that by-portion risk evaluation data are output according to an embodiment of the present disclosure.
FIG. 5 is a diagram illustrating that a 2-D pressure image is generated as 3-D data according to an embodiment of the present disclosure.
FIG. 6 is a diagram illustrating a method of evaluating a bedsore risk according to an embodiment of the present disclosure.
FIG. 7 is a diagram illustrating a method of estimating a body portion according to an embodiment of the present disclosure.

### DETAILED DESCRIPTION

The present disclosure is described hereinafter in detail with reference to the accompanying drawings. However, the present disclosure may be implemented in various different ways, and is not limited to the embodiments described herein. Furthermore, in the drawings, in order to clearly describe the present disclosure, parts unrelated to the description are omitted, and similar reference numbers are used to refer to similar parts throughout the specification.

In the entire specification, when it is described that one part is "connected to (or coupled with or brought into contact with or combined with)" the other part, the one part may be "directly connected" to the other part or may be "indirectly connected" to the other part through a third part. Furthermore, when it is said that any part "includes" a component, this means that the word "include" may further include another component not the exclusion of another component unless explicitly described to the contrary.

The terms used in this specification are used to only describe specific embodiments and are not intended to restrict the present disclosure. An expression of the singular number should be construed as including an expression of the plural number unless clearly defined otherwise in the context. It is to be understood that in this specification, a term, such as "include (or comprise)" or "have", is intended to designate the presence of a characteristic, a number, a step, an operation, a component, a part or a combination of them described in the specification and does not exclude the possible existence or addition of one or more other characteristics, numbers, steps, operations, components, parts or combinations of them in advance.

The term "module" used in this specification includes a unit configured as hardware, software or firmware, and may be interchangeably used with a term, such as logic, a logical block, a part or a circuit. The module may be an integrated part, a minimum unit to perform one or more functions, or a part thereof. For example, the module may be configured as an application-specific integrated circuit (ASIC).

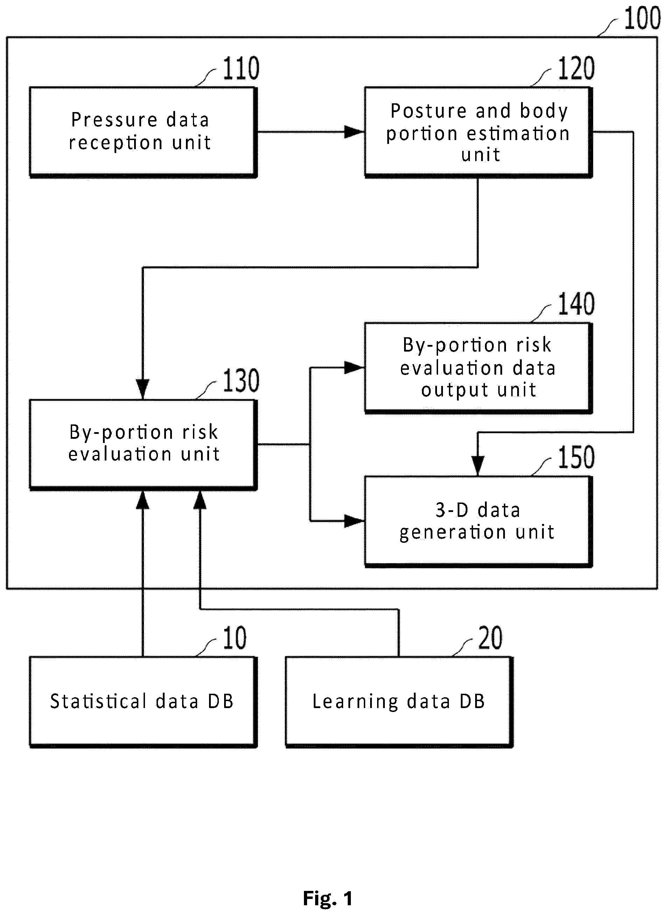

FIG. 1 is a diagram illustrating a construction of a system for evaluating a bedsore risk according to an embodiment of the present disclosure.

As illustrated FIG. 1, a system 100 for evaluating a bedsore risk may include a pressure data reception unit 110, a posture and body portion estimation unit 120, a by-portion risk evaluation unit 130, a by-portion risk evaluation data output unit 140, and a three-dimensional (3-D) data generation unit 150.

The pressure data reception unit 110 may receive pressure data from a pressure sensor (not illustrated). In this case, the pressure data reception unit 110 may receive the pressure data for each preset time interval. For example, the pressure data may have a form of a two-dimensional (2-D) image.

Furthermore, the pressure sensor may be constructed in a module type in which a plurality of pressure sensors has been divided. That is, the pressure sensor may measure pressure that is applied to each pressure sensor, and may transmit a measured pressure value to the pressure data reception unit 110.

The posture and body portion estimation unit 120 may estimate a posture and a body portion based on pressure data. In this case, a posture estimation model and a body portion estimation model may be stored in the posture and body portion estimation unit 120.

For example, the posture estimation model may estimate a posture and detect key points by using pressure data as an input. The key point refers to a preset feature point of a body. The posture estimation model may calculate a depth based on the pressure intensity of the pressure data. The posture estimation model may detect the coordinates of the key points in a 3-D space based on the calculated depth, may estimate a posture in a 2-D pressure image, and may output the posture.

The body portion estimation model may output the coordinate data of a pressure region of interest (PROI) by using, as an input, the coordinates of key points and a posture that are output by the posture estimation model. The PROI is an interested pressure region, and refers to a portion for which a bedsore risk is to be evaluated. In this case, the PROI may be differently constructed for each posture. The body portion estimation model may output the coordinate data of the PROI that is matched with a posture, based on the coordinates of the key points and the posture. For example, a clipping mask may be matched with the coordinates of an activated key point. As will be described later, the body portion estimation model may extract the coordinate data of the PROI by projecting a clipping mask onto a 2-D pressure image on the basis of the coordinates of the activated key point. Furthermore, the body portion estimation model may estimate a body portion based on the extracted coordinate data of the PROI.

The by-portion risk evaluation unit 130 may evaluate a degree of risk for each body portion, based on the coordinate data of the PROI extracted by the body portion estimation model, statistical data, and learning data. In this case, the by-portion risk evaluation unit 130 may receive the statistical data from a statistical data database (DB) 10. For example, the statistical data may include demographic data, such as sex, an age, a vital sign, and a timestamp. Furthermore, the by-portion risk evaluation unit 130 may receive the learning data from a learning data DB 20. The learning data may include a bedsore risk according to a body portion.

Furthermore, the by-portion risk evaluation unit 130 may assign an accumulation weight for each time. For example, the by-portion risk evaluation unit 130 may evaluate a degree of risk for each portion by assigning a weight to various parameters, such as an accumulated time, the presence or absence of bedsores, a nutrition state, and a skin state.

The by-portion risk evaluation data output unit 140 may receive by-portion risk evaluation data from the by-portion risk evaluation unit 130 and output the by-portion risk evaluation data. For example, the by-portion risk evaluation data output unit 140 may output by-portion risk evaluation data over time. In this case, the by-portion risk evaluation data may be divided into a first grade to a fourth grade, that is, steps in each of which the behavior of a person who takes action according to a bedsore occurrence risk is required. The by-portion risk evaluation data may be set to require a proactive behavior of a person who takes action as the number of the grade is increased.

The 3-D data generation unit 150 may generate a 2-D pressure image as 3-D data and provide the 3-D data. For example, the 3-D data generation unit 150 may generate a 2-D pressure image as 3-D data by receiving the coordinate data of a PROI from the posture and body portion estimation unit 120. Furthermore, the 3-D data generation unit 150 may receive by-portion risk evaluation data from the by-portion risk evaluation unit 130 and incorporate the by-portion risk evaluation data into the 3-D data. Accordingly, the 3-D data generation unit 150 can intuitively provide a degree of risk for each portion of an estimated posture to a person who takes action.

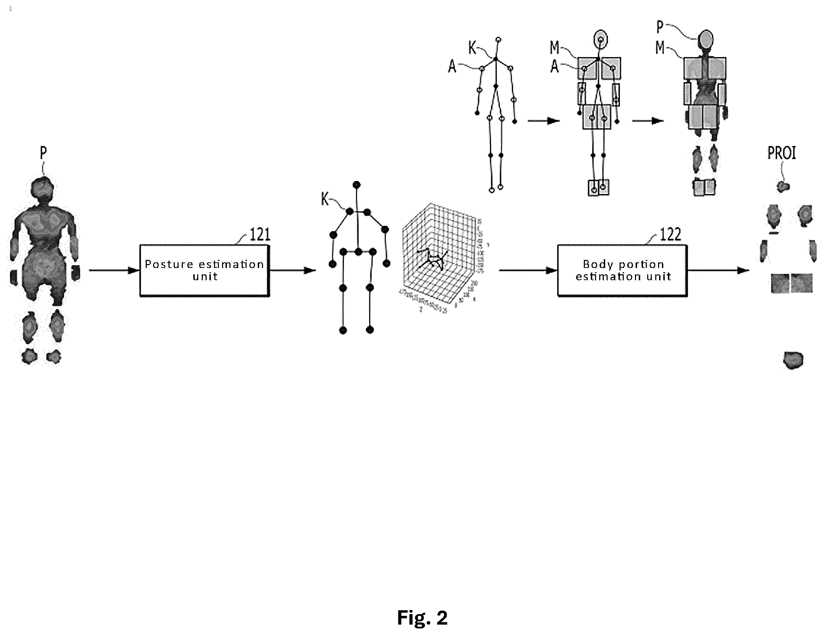

FIG. 2 is a diagram illustrating an operation of the posture and body portion estimation unit according to an embodiment of the present disclosure.

Referring to FIG. 2, the posture and body portion estimation unit may include a posture estimation unit 121 and a body portion estimation unit 122. The posture estimation unit 121 may output the coordinates of key points and a posture by using pressure data P as an input through the posture estimation model stored in the posture estimation unit 121. In this case, the key point refers to a preset feature point of a body. For example, the key points may include a head, elbows, shoulders, hips, and ankles. Furthermore, the pressure data P may consist of a 2-D pressure image. The posture estimation unit 121 may detect the coordinates of key points in a 3-D space by calculating a depth based on the pressure intensity of the pressure data P, and may estimate a posture in a 2-D pressure image.

Furthermore, the body portion estimation unit 122 may store the body portion estimation model. The body portion estimation unit 122 may output the coordinate data of a pressure region of interest (PROI) by using, as an input, the coordinates of key points and a posture that are output by the posture estimation unit 121.

First, the body portion estimation unit 122 may activate a key point K having coordinates, which is required for each posture. The body portion estimation unit 122 may generate a clipping mask on the basis of the coordinates of the activated key point A. In this case, the clipping mask is used to cut and use only a desired part of a specific image when a first layer enters a second layer region. The body portion estimation unit 122 may output the coordinate data of a PROI by projecting the generated clipping mask onto the pressure data P. The body portion estimation unit 122 may estimate a body portion according to a posture in the pressure data P based on the output coordinate data of the PROI. In this case, the body portion estimation unit 122 may adjust the size of a clipping mask M, based on the height and weight of a subject. That is, the body portion estimation unit 122 can check an interested pressure region into which body characteristics of a subject have been incorporated by generating a personalized clipping mask M.

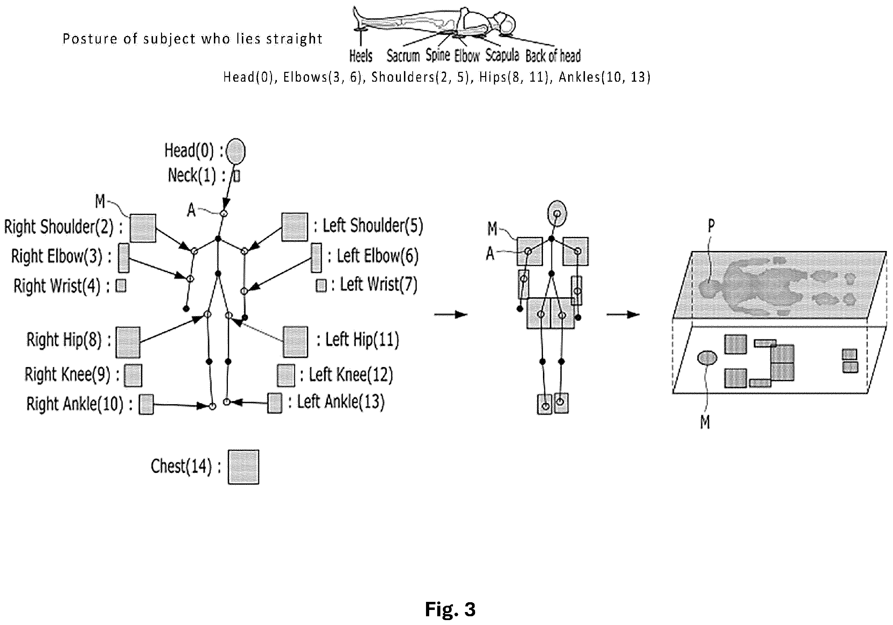

FIG. 3 is a diagram illustrating that the body portion estimation unit outputs the coordinate data of a PROI according to an embodiment of the present disclosure.

Referring to FIG. 3, the body portion estimation unit may generate the clipping mask M on the basis of the coordinates of the activated key point A. The body portion estimation unit may activate a key point having coordinates, which is required for each posture. For example, when a posture of a subject who lies straight is estimated, key points having coordinates, such as a head 0, elbows 3 and 6, shoulders 2 and 5, hips 8 and 11, and ankles 10 and 13, may be activated. The body portion estimation unit may generate the clipping mask M and match the clipping mask M with only the activated key point A.

Furthermore, the body portion estimation unit may output the coordinate data of a PROI by projecting the generated clipping mask M onto the pressure data P. As only a desired portion of the clipping mask is cut and use as described above, the body portion estimation unit may extract an interested pressure region according to a posture from the pressure data P by using the clipping mask. In this case, the coordinate data of the PROI may include coordinate data corresponding to a clipping mask region. Accordingly, the body portion estimation unit may estimate a body portion corresponding to the posture based on the coordinate data of the PROI.

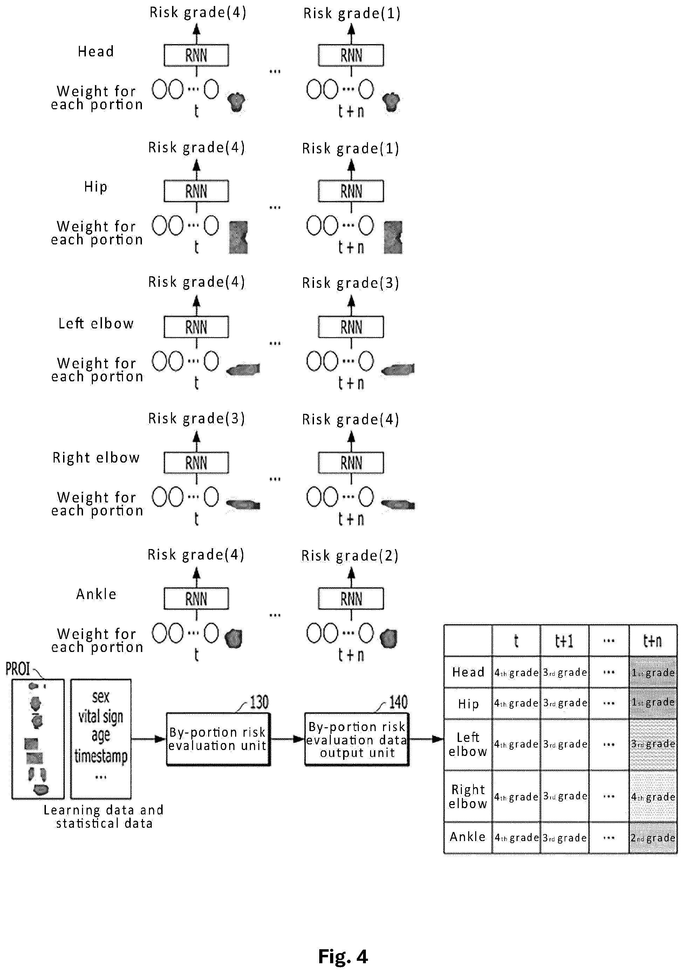

FIG. 4 is a diagram illustrating that by-portion risk evaluation data are output according to an embodiment of the present disclosure.

As illustrated in FIG. 4, the by-portion risk evaluation unit 130 may receive the coordinate data of a PROI, statistical data, and learning data. The statistical data may include demographic data, such as sex, an age, a vital sign, and a timestamp that are stored in the statistical data DB. Furthermore, the learning data may include a bedsore risk according to a body portion stored in the learning data DB. Accordingly, the by-portion risk evaluation unit 130 may evaluate a degree of risk based on conditions of a subject.

The by-portion risk evaluation unit 130 may evaluate a degree of risk for each portion based on the received coordinate data of the PROI, statistical data, and learning data. The by-portion risk evaluation unit 130 may use the coordinate data of the PROI that are received at specific time intervals. Referring to FIG. 4, the coordinate data of the PROI may include information on body portions, such as a head, hips, a left elbow, a right elbow, and ankles. That is, the by-portion risk evaluation unit 130 may evaluate a degree of risk for each body portion corresponding to the coordinate data of the PROI over time.

Furthermore, the by-portion risk evaluation unit 130 may assign an accumulation weight for each time. For example, the by-portion risk evaluation unit 130 may assign weights to various parameters, such as an accumulated time, the presence or absence of bedsores, a nutrition state, and a skin state. Accordingly, the by-portion risk evaluation unit 130 can evaluate a degree of risk for each portion more accurately by assigning a weight for each body portion.

The by-portion risk evaluation data output unit 140 may receive by-portion risk evaluation data from the by-portion risk evaluation unit 130 and output the by-portion risk evaluation data. For example, the by-portion risk evaluation data output unit 140 may divide the by-portion risk evaluation data into a first grade to a fourth grade, that is, steps in each of which the behavior of a person who takes action according to a bedsore occurrence risk is required. Furthermore, the by-portion risk evaluation data output unit 140 may provide the by-portion risk evaluation data over time in the form of a table. As illustrated in FIG. 4, a risk evaluation grade over time for each body portion may be displayed in the output by-portion risk evaluation data.

In this case, the first grade refers to the state in which the bedsore occurrence risk is very high and a person who takes action is required to take immediate measures. The second grade means that the bedsore occurrence risk is high, and refers to the state in which a person who takes action is required to perform consistent monitoring. The third grade means that the bedsore occurrence risk is middle, and refers to the state in which the attention of a person who takes action is required. The fourth grade refers to the state in which the bedsore occurrence risk is low and relatively safe. Furthermore, the by-portion risk evaluation data may be set to require a proactive behavior of a person who takes action as the number of the grade is increased. Furthermore, the by-portion risk evaluation data output unit 140 may assign a color to each grade so that a person who takes action can intuitively check the risk evaluation data.

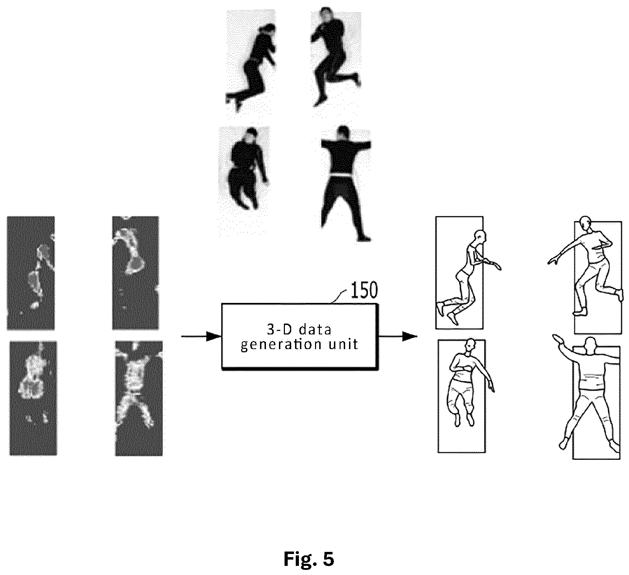

FIG. 5 is a diagram illustrating that a 2-D pressure image is generated as 3-D data according to an embodiment of the present disclosure.

The 3-D data generation unit 150 may generate input pressure data as 3-D data and provide the 3-D data. For example, the 3-D data generation unit 150 may generate a 2-D pressure image as 3-D data based on the coordinate data of a PROI including body portion information according to a posture. In this case, the 3-D data generation unit 150 may receive the coordinate data of the PROI corresponding to the 2-D pressure image from the posture and body portion estimation unit. The 3-D data generation unit 150 can generate the 2-D pressure image as the 3-D data more accurately by incorporating the body portion information according to an identified posture.

Furthermore, the 3-D data generation unit 150 may supplement the generated 3-D data based on pre-stored posture image data. A 2-D pressure image is information on pressure that is applied to the pressure sensor by the body of a subject. Accordingly, a portion of the body with which the pressure sensor does not come into contact is not accurately displayed in the 2-D pressure image because pressure data of the portion is not measured. Accordingly, the 3-D data generation unit 150 can generate 3-D data more accurately by incorporating posture image data corresponding to the coordinate data of a PROI.

Furthermore, the 3-D data generation unit 150 may receive by-portion risk evaluation data and incorporate the by-portion risk evaluation data into the 3-D data. Accordingly, the 3-D data generation unit 150 can intuitively provide a degree of risk for each portion of an estimated posture to a person who takes action.

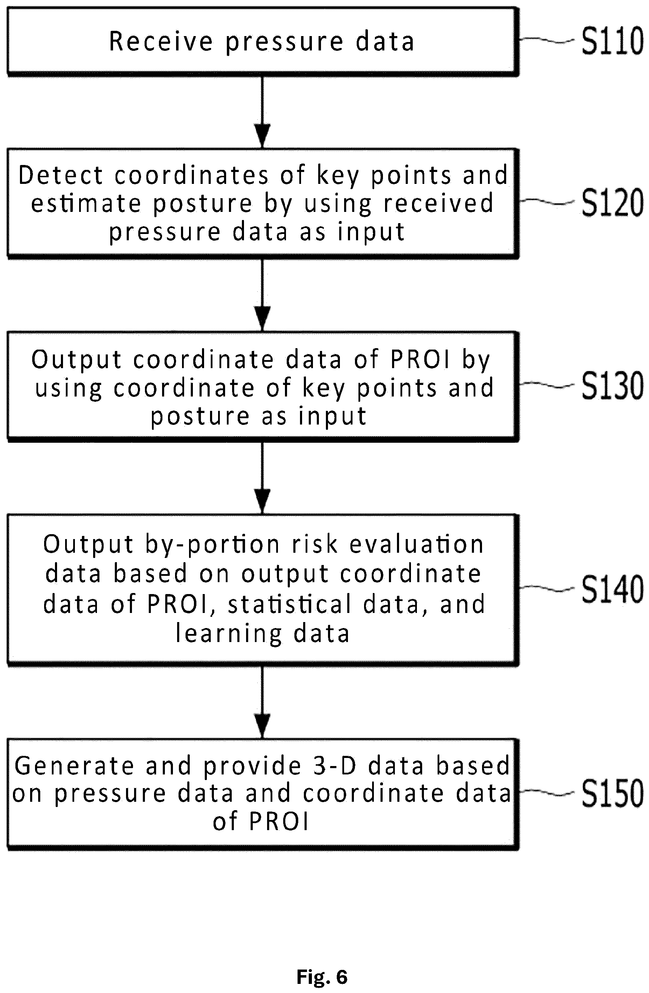

FIG. 6 is a diagram illustrating a method of evaluating a bedsore risk according to an embodiment of the present disclosure.

In step S110, the system for evaluating a bedsore risk may receive pressure data from the pressure sensor. The system for evaluating a bedsore risk may receive the pressure data having a 2-D image form every preset time.

In step S120, the system for evaluating a bedsore risk may detect the coordinates of key points and estimate a posture by using the received pressure data as an input. For example, the system for evaluating a bedsore risk may detect the coordinates of the key points in a 3-D space by calculating a depth based on the pressure intensity of the pressure data, and may estimate the posture in a 2-D pressure image. In this case, the key point refers to a preset feature point of a body. For example, the key points may include feature points of a body, such as a head, elbows, shoulders, hips, and ankles. Accordingly, the system for evaluating a bedsore risk can estimate the posture by extracting the key points.

In step S130, the system for evaluating a bedsore risk may output the coordinate data of a pressure region of interest (PROI) by using the coordinates of the key points and the posture as an input. The PROI is an interested pressure region, and refers to a portion for which a bedsore risk is to be evaluated. In this case, the PROI may be differently constructed for each posture.

Furthermore, the system for evaluating a bedsore risk may activate a key point having coordinates, which is required for each posture. The key point having the coordinates, which is required for each posture, may be preset. Furthermore, the system for evaluating a bedsore risk may generate and match a clipping mask on the basis of the activated key point. In this case, the clipping mask may be used to cut and use only a desired portion of a specific image when a first layer enters a second layer region. Accordingly, the system for evaluating a bedsore risk may extract a 2-D pressure image corresponding to the clipping mask as an interested pressure region. Furthermore, the system for evaluating a bedsore risk may output coordinate data corresponding to the extracted 2-D pressure image as the coordinate data of a PROI. In this case, the system for evaluating a bedsore risk may estimate a body portion according to a posture based on the output coordinate data of the PROI.

In step S140, the system for evaluating a bedsore risk may evaluate a degree of risk for each body portion based on the extracted coordinate data of the PROI, statistical data, and learning data. In this case, the system for evaluating a bedsore risk may receive the statistical data from the statistical data DB. Furthermore, the system for evaluating a bedsore risk may receive the learning data from the learning data DB. The statistical data DB and the learning data DB may be stored in the system for evaluating a bedsore risk. Furthermore, the statistical data DB and the learning data DB may be stored outside and provided in response to a request from the system for evaluating a bedsore risk. For example, the statistical data may include demographic data, such as sex, an age, a vital sign, and a timestamp. Furthermore, the learning data may include a bedsore risk according to a body portion.

Furthermore, the system for evaluating a bedsore risk may assign an accumulation weight for each time. For example, the system for evaluating a bedsore risk may evaluate a degree of risk for each portion by assigning weights to various parameters, such as an accumulated time, the presence or absence of bedsores, a nutrition state, and a skin state. Furthermore, the system for evaluating a bedsore risk may output by-portion risk evaluation data over time. In this case, the by-portion risk evaluation data may be divided into a first grade to a fourth grade, that is, steps in each of which the behavior of a person who takes action according to a bedsore occurrence risk is required. The by-portion risk evaluation data may be set to require a proactive behavior of a person who takes action as the number of the grade is increased.

In step S150, the system for evaluating a bedsore risk may generate and provide 3-D data based on the pressure data and the coordinate data of the PROI. The system for evaluating a bedsore risk may generate 2-D pressure image as the 3-D data based on the coordinate data of the PROI including body portion information according to a posture. The system for evaluating a bedsore risk can generate the 2-D pressure image as the 3-D data more accurately by incorporating the body portion information according to an identified posture.

FIG. 7 is a diagram illustrating a method of estimating a body portion according to an embodiment of the present disclosure.

In step S131, the system for evaluating a bedsore risk may input the coordinates of key points and a posture to the body portion estimation model. The system for evaluating a bedsore risk may store the body portion estimation model that can estimate a body portion by detecting the coordinate data of a PROI by using the coordinates of the key points and the posture as an input.

In step S132, the system for evaluating a bedsore risk may activate a preset key point having coordinates, which is required for each posture. For example, the system for evaluating a bedsore risk may activate the coordinates of key points corresponding to a head, elbows, shoulders, hips, and ankles in a posture of a subject who lies straight.

In step S133, the system for evaluating a bedsore risk may generate a clipping mask on the basis of the coordinates of the activated key point. In this case, the system for evaluating a bedsore risk may adjust the size of the clipping mask based on body conditions, such as the height and weight of a subject. That is, the system for evaluating a bedsore risk can identify a body portion more accurately by personalizing the clipping mask.

In step S134, the system for evaluating a bedsore risk may extract the coordinate data of a PROI by projecting the generated clipping mask onto the pressure data. The coordinate data of the PROI may include coordinate information of the pressure data corresponding to the clipping mask. The coordinate data of the PROI may be preset based on the posture. Accordingly, the system for evaluating a bedsore risk may estimate the body portion for each posture based on the extracted coordinate data of the PROI.

The description of the present disclosure is illustrative, and a person having ordinary knowledge in the art to which the present disclosure pertains will understand that the present disclosure may be easily modified in other detailed forms without changing the technical spirit or essential characteristic of the present disclosure. Accordingly, it should be construed that the aforementioned embodiments are only illustrative in all aspects, and are not limitative. For example, components described in the singular form may be carried out in a distributed form. Likewise, components described in a distributed form may also be carried out in a combined form.

The scope of the present disclosure is defined by the appended claims rather than by the detailed description, and all changes or modifications derived from the meanings and scope of the claims and equivalents thereto should be interpreted as being included in the scope of the present disclosure.

## Claims

1. A system for evaluating a bedsore risk, comprising:
a pressure data reception unit configured to receive pressure data from a plurality of pressure sensors;
a posture and body portion estimation unit configured to detect coordinates of key points that are feature points of a body and estimate a posture based on pressure data, to output coordinate data of a pressure region of interest (PROI) based on the coordinates of the key points and the posture, and to estimate a body portion;
a by-portion risk evaluation unit configured to evaluate a degree of risk for each body portion based on the coordinate data of the PROI, statistical data, and learning data and to generate by-portion risk evaluation data; and
a by-portion risk evaluation data output unit configured to output the by-portion risk evaluation data by dividing the by-portion risk evaluation data into grades according to bedsore occurrence risks.

2. The system of claim 1, wherein the posture and body portion estimation unit comprises:
a posture estimation unit configured to store a posture estimation model that outputs the coordinates of the key points and the posture by using the pressure data as an input; and
a body portion estimation unit configured to store a body portion estimation model that outputs the coordinate data of the PROI by using the coordinates of the key points and the posture as an input.

3. The system of claim 2, wherein the posture estimation model calculates a depth based on a pressure intensity of the pressure data and detects the coordinates of the key points in a three-dimensional (3-D) space based on the calculated depth.

4. The system of claim 2, wherein the body portion estimation model
activates the key point having coordinates, which is required for each posture, and generates a clipping mask on the basis of the coordinates of the activated key point, and
extracts the coordinate data of the PROI by projecting the generated clipping mask onto the pressure data.

5. The system of claim 1, wherein:
the by-portion risk evaluation unit outputs the by-portion risk evaluation data by assigning an accumulation weight over time to the by-portion risk evaluation data, and
the accumulation weight comprises at least one of an accumulated time, a presence or absence of bedsores, a nutrition state, or a skin state.

6. The system of claim 1, further comprising a 3-D data generation unit configured to generate and provide the pressure data as 3-D data based on the coordinate data of the PROI,
wherein the generated 3-D data are supplemented by using pre-stored posture image data and are displayed by incorporating the by-portion risk evaluation data.

7. A method of evaluating a bedsore risk, comprising steps of:
a) receiving pressure data from a plurality of pressure sensors;
b) detecting coordinates of key points that are feature points of a body and estimating a posture by inputting the pressure data to a posture estimation model;
c) outputting coordinate data of a pressure region of interest (PROI) that is an interested pressure region by inputting the coordinates of the key points and the posture to a body portion estimation model and estimating a body portion; and
d) evaluating a degree of risk for each body portion and generating by-portion risk evaluation data based on the coordinate data of the PROI, statistical data, and learning data and outputting the by-portion risk evaluation data by dividing the by-portion risk evaluation data into grades according to bedsore occurrence risks.

8. The method of claim 7, wherein the step b) comprises:
calculating a depth based on a pressure intensity of the pressure data, and
detecting the coordinates of the key points in a three-dimensional (3-D) space based on the calculated depth.

9. The method of claim 7, wherein the step c) comprises:
activating a key point having coordinates, which is required for each posture;
generating a clipping mask on the basis of coordinates of the activated key point; and
extracting the coordinate data of the PROI by projecting the generated clipping mask onto the pressure data.

10. The method of claim 1, wherein:
the step d) comprises outputting the by-portion risk evaluation data by assigning an accumulation weight over time to the by-portion risk evaluation data, and
the accumulation weight comprises at least one of an accumulated time, a presence or absence of bedsores, a nutrition state, or a skin state.

11. The method of claim 7, further comprising a step e) of generating and providing pressure data as 3-D data based on the coordinate data of the PROI,
wherein the step e) comprises:
supplementing the generated 3-D data by using pre-stored posture image data, and
displaying the supplemented 3-D data by incorporating the by-portion risk evaluation data into the supplemented 3-D data.
